# EUROPEAN PATENT SPECIFICATION

(11) **EP 2 802 319 B1**
(45) Date of publication and mention of the grant of the patent: **25.10.2017**
(21) Application number: 13736198.6
(22) Date of filing: 10.01.2013
(51) Int. Cl.: A61K 31/135, A61K 31/136, A61K 31/428, A61K 9/00, A61K 9/20, A61K 9/50, A61K 45/06, A61P 25/16

(54) **FIXED DOSE COMBINATION THERAPY OF PARKINSON'S DISEASE**
FESTDOSIS-KOMBINATIONSTHERAPIE VON MORBUS PARKINSON
POLYTHÉRAPIE À BASE DE DOSES FIXES POUR LE TRAITEMENT DE LA MALADIE DE PARKINSON

(30) Priority: 12.01.2012 US 201261585824 P
(43) Date of publication of application: 19.11.2014
(73) Proprietor: Pharma Two B Ltd., 76702 Rehovot (IL)
(72) Inventor: LIVNAH, Nurit, 76804 Mazkeret Batya (IL); LITMAN, Pninit, 74045 Ness Ziona (IL); ZAKSH, Sarit, 71704 Modiin (IL)
(74) Representative: Gevers & Orès
(86) International application number: PCT/IL2013/050025
(87) International publication number: WO 2013/105092

(56) References cited:
- WO-A1-2008/129043
- WO-A1-2009/147681
- WO-A1-2010/022140
- WO-A1-2011/095973
- WO-A2-2006/014973
- US-A1- 2007 232 700
- PIZARRO M ET AL: "PND20 Cost Effectiveness of Rasagiline and Pramipexol in Early Parkinson's Disease in the Mexican Setting", VALUE IN HEALTH, vol. 15, no. 4, 2012, XP028924444, ISSN: 1098-3015, DOI: 10.1016/J.JVAL.2012.03.779
- H. REICHMANN: 'Initiation of Parkinson's disease treatment' JOURNAL OF NEUROLOGY vol. 255, 05 September 2008, pages 57 - 59, XP055080587
- HAUSER R.A. ET AL.: 'Advances in the pharmacologic management of early Parkinson disease' NEUROLOGIST vol. 13, no. 3, May 2007, pages 126 - 132, XP008174367

## Description

### FIELD OF THE INVENTION

The present invention is in the field of neurodegenerative diseases and, in particular, relates to compositions for use in the treatment of Parkinson's disease.

### BACKGROUND OF THE INVENTION

Dopamine Agonists are commonly used in the treatment of Parkinson's disease; however, their use can be limited by adverse events with various levels of severity. The initiation of dopamine agonists are typically associated with nausea, vomiting and orthostatic hypotension. These side effects are more pronounced with higher doses but can usually be mitigated with a slow and complex titration schedule. Pramipexole (as well as several other DA agonists) is also associated with impulse control disorders, peripheral edema, psychosis, and sedation, that can be difficult to control and therefore limit the utility of this medication. rasagiline, another drug used in the treatment of Parkinson's disease, is largely well tolerated but also has some safety concerns particularly with respect to the risk of a cheese reaction (hypertensive crisis) with foods that are high in tyramine and a serotonin reaction (excess serotonin activity) when employed in combination with selective serotonin reuptake inhibitors and other anti-depressants that are commonly prescribed in Parkinson's disease. Among both drugs higher doses of pramipexole are typically associated with a greater risk of sever adverse effects and therefore it is important for clinicians to have treatment strategies that allow for the greatest efficacy in controlling PD symptoms, while minimizing motor complications and DA-induced adverse events.

Pharma Two B discovered that combining agents with complementary mechanisms of action (i.e., two different active agents having symptomatic or neuroprotective effects) allows for enhanced anti-Parkinsonian efficacy in comparison to what can be achieved with higher doses of either agent alone (WO2009147681). Preclinical data generated previously by Pharma Two B suggests that low doses of the MAO-B inhibitor rasagiline and the dopamine agonist pramipexole act synergistically in improving the effectiveness of these drugs. Given the relatively troublesome adverse event profile associated with initiating treatment with dopamine agonists, current treatments include a titration schedule of low doses, which are not expected to have therapeutic effect but to minimize the side effects caused by immediate start with effective doses. Moreover, undesirable effects are associated with long-term treatment with higher doses of the dopamine agonist. Thus, the option of using a combination containing low doses of dopamine agonist is favorable for many patients and may provide high therapeutic effect with minimal side effects.

### SUMMARY OF INVENTION

In some aspects, the present invention provides a pharmaceutical composition for use in treatment of Parkinson's disease comprising a pharmaceutically acceptable carrier and a fixed dose combination of pramipexole and rasagiline, wherein the fixed dose combination contains from 0.05 mg to 1.0 mg of pramipexole and from 0.05 mg to 1.0 mg of rasagiline, and the dose of pramipexole is lower than the dose of rasagiline.

In another aspect, the present invention provides methods for preparing an extended release (ER) formulation of a fixed dose combination of pramipexole and rasagiline, or a pharmaceutically acceptable salt thereof, said method comprising the steps of:
(i) dissolving an active agent comprising pramipexole, rasagiline or both, optionally suitably admixed with a binder and/or a glidant, in a suitable solvent system to prepare a uniform suspension;
(ii) applying a coat of the suspension obtained in (i) to inert pellets such as inert nonpareil seeds;
(iii) optionally coating the rasagiline loaded pellets, pramipexole-loaded pellets or pellets loaded with both pramipexole and rasagiline, obtained in (ii) with an insulating/protecting sub-coating layer;
(iv) coating the pellets obtained in (ii) or (iii) with an extended-release coating layer which enables an extended release of said pramipexole and rasagiline thereby obtaining said extended release formulation;
(v) optionally blending the coated pellets obtained in (iv) with a suitable excipient; and
(vi) filling said extended release formulation into capsules or compressing said extended release formulation into tablets, wherein said capsules or tablets comprise a ratio of pramipexole-loaded pellets and rasagiline-loaded pellets selected from a range of 1:1.1 to 1:20, 1:1.1 to 1:1, 1:1.1 to 1:5, 1:1.1 to 1:3 or 1:1.1 to 1:2; or said capsules or tablets comprise pellets loaded with both pramipexole and rasagiline at a ratio selected from a range of 1:1.1 to 1:20, 1:1.1 to 1:10, 1:1.1 to 1:5, 1:1.1 to 1:3 or 1:1.1 to 1:2,
thereby obtaining an extended release formulation of a fixed dose combination of pramipexole and rasagiline.

### BRIEF DESCRIPTION OF DRAWINGS

**Fig. 1** shows the dissolution profile of the combination product.
**Fig. 2** shows dose dependent synergistic effect of pramipexole, rasagiline and their combination, on dopamine levels in mouse brain.
**Figs. 3A-B** depict a pharmacokinetic study of P2B001 (FDC containing 1mg rasagiline and 0.75 mg pramipexole) in comparison to the respective commercial drugs, Azilect (1mg rasagiline) and Mirapex ER (0.75mg pramipexole), given alone or in combination. Concentration in plasma (pg/ml) of pramipexole and rasagiline

### DETAILED DESCRIPTION OF THE INVENTION

The present invention is based on the finding that it is possible to use very low doses of dopamine agonist, particularly doses that are currently not typically used as monotherapy and are typically used for titration, combined with various doses of the monoamine oxidase B (MAOB) inhibitor rasagiline, and receive high efficacy, due to the synergy between the mechanisms of actions of the two drugs.

This finding enables us to decrease the doses of pramipexole and thus avoid the risk of dopamine agonist (DA) -induced adverse events, while maintaining comparable efficacy in the patients. In that way it is possible to determine more precisely the best doses of the combination of rasagiline and pramipexole that is associated with a meaningful anti-Parkinson effect and a good safety profile.

Thus, according to the present invention, a fixed dose combination containing from 0.05 mg to 1.0 mg of pramipexole and from 0.05 mg to 1.0 mg of rasagiline, or a pharmaceutically acceptable salt thereof, wherein pramipexole is present at a dose lower than the dose of rasagiline, is efficacious in treating Parkinson's disease.

The term "Fixed Dosage Combination" as used herein refers to a single dosage formulation comprising two different drugs, in this case rasagiline and pramipexole, at a precise ratio, namely, in certain fixed doses.

The term "subtherapeutic dose" as used herein refers to a dose that is below the effective monotherapy dosage levels commonly used to treat a disease, or a dose that currently is not typically used for effective monotherapy, i.e. about 1 mg/day in the case of both pramipexole and rasagiline.

In certain embodiments, the molar ratio of pramipexole to rasagiline is selected from a range of 1:1.1 to 1:20, 1:1.1 to 1:10, 1:1.1 to 1:5, 1:1.1 to 1:3 or 1:1.1 to 1:2. In particular, this ratio is selected from a group consisting of 1:1.1, 1:1.2, 1:1.3, 1:1.4, 1:1.5, 1:1.6, 1:1.7, 1:1.8, 1;1.9, 1:2.0, 1:2.1, 1:2.2, 1:2.3, 1:2.4, 1:2.5, 1:2.6, 1:2.7, 1:2.8, 1:2.9 and 1:3.0.

In certain embodiments, the fixed dose combination contains between 0.1 and 0.6 mg pramipexole and between 0.1 to 0.75 mg rasagiline.

In certain embodiments, the fixed dose combination may contain 0.05, 0.055, 0.06, 0.065, 0.07, 0.075, 0.08, 0.085, 0.09, 0.095, 0.1, 0.105, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195, 0.2, 0.205, 0.21, 0.215, 0.22, 0.225, 0.23, 0.235, 0.24, 0.245, 0.25, 0.255, 0.26, 0.265, 0.27, 0.275, 0.28, 0.285, 0.29, 0.295, 0.3, 0.305, 0.31, 0.315, 0.32, 0.325, 0.33, 0.335, 0.34, 0.345, 0.35, 0.355, 0.36, 0.365, 0.37, 0.375, 0.38, 0.385, 0.39, 0.395, 0.4, 0.405, 0.41, 0.415, 0.42, 0.425, 0.43, 0.435, 0.44, 0.445, 0.45, 0.455, 0.46, 0.465, 0.47, 0.475, 0.48, 0.485, 0.49, 0.495, 0.5, 0.505, 0.51, 0.515, 0.52, 0.525, 0.53, 0.535, 0.54, 0.545, 0.55, 0.555, 0.56, 0.565, 0.57, 0.575, 0.58, 0.585, 0.59, 0.595, 0.6, 0.605, 0.61, 0.615, 0.62, 0.625, 0.63, 0.635, 0.64, 0.645, 0.65, 0.655, 0.66, 0.665, 0.67, 0.675, 0.68, 0.685, 0.69, 0.695, 0.7, 0.705, 0.71, 0.715, 0.72, 0.725, 0.73, 0.735, 0.74, 0.745, 0.75, 0.755, 0.76, 0.765, 0.77, 0.775, 0.78, 0.785, 0.79, 0.795, 0.8, 0.805, 0.81, 0.815, 0.82, 0.825, 0.83, 0.835, 0.84, 0.845, 0.85, 0.855, 0.86, 0.865, 0.87, 0.875, 0.88, 0.885, 0.89, 0.895, 0.9, 0.905, 0.91, 0.915, 0.92, 0.925, 0.93, 0.935, 0.94, 0.945, 0.95, 0.955, 0.96, 0.965, 0.97, 0.975, 0.98, 0.985, 0.99, 0.995 or 1 mg of pramipexole; and 0.05, 0.055, 0.06, 0.065, 0.07, 0.075, 0.08, 0.085, 0.09, 0.095, 0.1, 0.105, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195, 0.2, 0.205, 0.21, 0.215, 0.22, 0.225, 0.23, 0.235, 0.24, 0.245, 0.25, 0.255, 0.26, 0.265, 0.27, 0.275, 0.28, 0.285, 0.29, 0.295, 0.3, 0.305, 0.31, 0.315, 0.32, 0.325, 0.33, 0.335, 0.34, 0.345, 0.35, 0.355, 0.36, 0.365, 0.37, 0.375, 0.38, 0.385, 0.39, 0.395, 0.4, 0.405, 0.41, 0.415, 0.42, 0.425, 0.43, 0.435, 0.44, 0.445, 0.45, 0.455, 0.46, 0.465, 0.47, 0.475, 0.48, 0.485, 0.49, 0.495, 0.5, 0.505, 0.51, 0.515, 0.52, 0.525, 0.53, 0.535, 0.54, 0.545, 0.55, 0.555, 0.56, 0.565, 0.57, 0.575, 0.58, 0.585, 0.59, 0.595, 0.6, 0.605, 0.61, 0.615, 0.62, 0.625, 0.63, 0.635, 0.64, 0.645, 0.65, 0.655, 0.66, 0.665, 0.67, 0.675, 0.68, 0.685, 0.69, 0.695, 0.7, 0.705, 0.71, 0.715, 0.72, 0.725, 0.73, 0.735, 0.74, 0.745, 0.75, 0.755, 0.76, 0.765, 0.77, 0.775, 0.78, 0.785, 0.79, 0.795, 0.8, 0.805, 0.81, 0.815, 0.82, 0.825, 0.83, 0.835, 0.84, 0.845, 0.85, 0.855, 0.86, 0.865, 0.87, 0.875, 0.88, 0.885, 0.89, 0.895, 0.9, 0.905, 0.91, 0.915, 0.92, 0.925, 0.93, 0.935, 0.94, 0.945, 0.95, 0.955, 0.96, 0.965, 0.97, 0.975, 0.98, 0.985, 0.99, 0.995, or 1 mg of rasagiline, provided that the dose of pramipexole is lower than the dose of rasagiline as defined above.

For purposes of clarity, the numerical parameters recited in the present specification are approximations that may vary depending on the desired outcome. For example, each numerical parameter may be construed in light of the number of reported significant digits and by applying ordinary rounding techniques.

Any dose range, amount range, concentration range, percentage range, or ratio range recited herein are to be understood to include doses, concentrations, percentages or ratios of any integer within that range, unless otherwise indicated.

In other embodiments, the pramipexole and rasagiline are formulated for extended release (ER). The term "extended release" is used herein interchangeably with the terms "prolonged-action", "repeat-action", "controlled release", and "sustained-release" and refers to the release of an active agent at predetermined intervals or gradually, in such a manner as to make the contained active agent available over an extended period of time following ingestion.

In certain embodiments, all or nearly all pramipexole and rasagiline in the fixed dose combination is gradually released from the extended release formulation over a period of 24 hours.

The pharmaceutical composition may be in the form of a monolithic matrix; a tablet, preferably a bi- or multi-layered tablet, matrix tablet, disintegrating tablet, dissolving tablet, or chewable tablet; a capsule or sachet, preferably filled with granules, grains, beads, or pellets; or a depot system based on a biodegradable polymer such as poly(D,L-lactide) (PLA), polyglycolide (PGA), and poly(D,L-lactide-co-glycolide) (PLGA), and it may be formulated for oral administration.

The active agent may be suitably admixed with a binder and/or a glidant, in a suitable solvent system to prepare a uniform suspension and then applied to inert pellets to form a thin coat. In the case of the present invention, rasagiline and pramipexole may be dissolved in separate solvents and sprayed separately on different pellets to form rasagiline loaded pellets and pramipexole-loaded pellets; or each separate solution may be sprayed on the same pellets to form pellets loaded with both rasagiline and pramipexole Alternatively, rasagiline and pramipexole may be dissolved in a common solvent to form a common uniform solution, or the separate solutions may be mixed to form a common uniform solution, and the common uniform solution may be sprayed on pellets to form pellets loaded with both rasagiline and pramipexole.

In the next step, which is optional, the rasagiline loaded pellets, the pramipexole-loaded pellets or the pellets loaded with both rasagiline and pramipexole, are coated with an insulating/protecting sub-coating layer, after which the pellets are coated with an extended-release coating layer which enables an extended release of the rasagiline and pramipexole thereby obtaining said extended release formulation. The coated pellets may then be blended with a suitable excipient, and finally the extended release formulation is filled into capsules or compressed into tablets, wherein said capsules or tables comprise a desired ratio of rasagiline loaded pellets and pramipexole-loaded pellets; or said capsules or tablets comprise pellets loaded with both rasagiline and pramipexole.

The desired ratio, as defined herein above, may be obtained using any method that will provide the desired result, such as weighing, measuring the volume of, or counting, the rasagiline loaded pellets and pramipexole-loaded pellets separately and filling the capsule, or compressing the tablet, with the desired weight, volume or number of each active agent-loaded pellet. Preferably, the pellets are weighed separately and filled at the desired ratio into capsules or pressed into tablets, or mixed together at a pre-determined ratio and the mix is weighed into the capsule. In the case of pellets loaded with both rasagiline and pramipexole, the ratio is determined at the stage of coating the inert pellets, in which a solution with the desired ratio of the two agents is spayed on the inert pellets, or two separate solutions are sprayed in layers on the inert pellets, at the desired ratio.

Thus, in certain embodiments, pharmaceutical composition of the present invention comprises extended-release pellets comprising (i) an inert pellet core; (ii) a drug layer coating said pellet core, said drug layer comprising an active agent comprising rasagiline, pramipexole or both, or a pharmaceutically acceptable salt thereof, optionally suitably admixed with a binder and/or a film-former polymer, and further optionally admixed with a glidant; (iii) optionally an isolating/protecting sub-coating layer coating said drug layer; and (iv) an extended-release coating layer coating said sub-coating layer, if present, or said drug layer.

The ER pellet of the present invention may optionally comprise an isolating/protecting sub-coating layer coating said drug layer. The role of this sub-coating layer is to isolate the active material layer from the external ER coating and protect from possible interactions with the active agent that might affect its stability and lead to formation of active pharmaceutical ingredient (API) degradation products. In certain embodiments, the sub-coating layer comprises a film-former polymer and optionally a glidant.

The ER pellet of the present invention comprises an outer ER coating layer, also termed herein "a functional layer", coating either the sub-coating layer, if present, or the drug layer.

In certain embodiments, the ER coating layer comprises at least one pH-independent polymer, i.e., a water swelling/water insoluble/hydrophobic polymer, and optionally a pore-forming agent, wherein the extended-release pellet has a pH-independent in vitro release characteristic. In other embodiments, the functional layer comprises a pH-independent polymer, a hydrophilic release modulator polymer acting as a pore-forming agent, and optionally a hydrophobic or hydrophilic plasticizer, and/or glidant. In further certain embodiments, the ER coating layer comprises a mixture of a pH-dependent enteric-coating polymer and a pH-independent polymer, wherein the extended-release pellet has a close to zero order in vitro release characteristic at either acidic or physiological pH, i.e., at pH values of up to 7.4.

Binders for pharmaceutical use are hydrophilic substances, such as sugars and polymers of natural and synthetic origin, used in the manufacture of solid dosage forms due to their adhesive and cohesive properties. The role of binders is to assist size enlargement by adding cohesiveness to powders, thereby providing granules and tablets with the necessary bonding strength. Although binders improve the appearance, hardness and friability of these preparations, they are not intended to influence the disintegration or dissolution rates of the active substances. Binders of natural origin, which have been commonly used in the past, include acacia, gelatin, starch, and hydrolyzed starch. Those substances have been replaced by binders of synthetic origin, the most important of which are povidone and various cellulose derivatives. Examples of binders that can be admixed with the active agent in the drug layer coating of the ER pellet of the invention include a polyvinyl pyrrolidone (PVP), hydroxypropylmethyl cellulose (HPMC), hydroxypropyl cellulose (HPC), microcrystalline cellulose, and combinations thereof. The binder may be present in an amount from 0.5% to 20%, preferably from 0.5% to 10%, by weight of the entire pellet.

The term "film-former polymer" as used herein refers to polymers capable of hardening to coherent films. In addition, the physical property of these polymers that is essential for coating is the ability to form films or certain adhesiveness to the material to be coated. Examples of film-former polymers include PVP, HPMC, HPC, microcrystalline cellulose, and combinations thereof. The film-former polymer when comprised within the drug layer may be present in an amount of up to 90% by weight of the entire drug layer, preferably from 0.5% to 20%, by weight of the entire pellet. The amount of film-former polymer in the sub-coating layer may be up to 100% by weight of the entire sub-coating layer, preferably from 0.5% to 10%, by weight of the entire pellet.

Glidants are typically added to pharmaceutical compositions to enhance flowability of granulations and powders by reducing friction and surface charge. In addition, they are used as anti-tack agents during the coating process. Particular glidants such as talc and glyceryl monostearate are commonly used in coating formulations as anti-tack agents, which reduce the sticking tendency at lower product temperatures. Other glidants such as silicon dioxide colloidal provide desirable flow characteristics that are exploited to improve the flow properties of dry powders in a number of processes such as tableting and capsulation, due to their small particle size and large specific surface area. Examples of glidants include talc, particularly talc extra fine, colloidal silicon dioxide, glyceryl monostearate, and combinations thereof.

The glidants, when comprised within the drug layer, may be present in an amount of up to 30% by weight of the entire drug layer, preferably from 0.5% to 5%, by weight of the entire pellet. The amount of glidant when comprised within the sub-coating layer may be up to 10% by weight of the entire sub-coating layer, preferably from 0.5% to 5%, by weight of the entire pellet.

Examples of pH-independent polymers that may be comprised within the ER pellet of the invention include ethyl cellulose, Surelease®, copolymers of acrylic and methacrylic acid esters such as Eudragit® RL (a copolymer of ethylacrylate, methylmethacrylate and trimethylammonioethyl methacrylate chloride), 1:2:0.2), Eudragit® RS (a copolymer of ethylacrylate, methyl methacrylate and trimethylammonioethyl methacrylate chloride, 1:2:0.1), Eudragit® NE (a copolymer of (ethylacrylate and methylmethacrylate, 2:1), and combinations thereof. The pH-independent polymer may be present in an amount from 10% to 50%, preferably from 10% to 30%, by weight of the entire pellet.

Examples of pH-dependent enteric-coating polymers that may be comprised within the ER pellet of the invention include Eudragit® S (poly(methacrylicacid, nnethylmethacrylate), 1:2), Eudragit® L 55 (poly (methacrylicacid, ethylacrylate), 1:1), Kollicoat® (polyvinyl alcohol-polyethylene glycol graft copolymer), 1:1), hydroxypropyl methylcellulose phthalate (HPMCP), alginates, carboxymethylcellulose, and combinations thereof. The pH-dependent enteric-coating polymer may be present in an amount from 10% to 50%, preferably from 10% to 30%, by weight of the entire pellet.

The term "pore-forming agent" as used herein refers to a substance that dissolves in the body environment, thus forming open pores in the matrix that increase the diffusion rate of the active agent through the coating layer. The size of the pores formed can, to some extent, be controlled by the size of the solid particulate material being used. For uniformity of pores, the particulate material can be screened through successively finer mesh sieves to produce a desired range of particle sizes. The pore-forming agent that may be comprised within the ER pellets of the invention is either inorganic or organic substance, including, e.g., polyvinylpyrrolidone (PVP), polyethylene glycol (PEG), HPMC, HPC, methylcellulose, 1,2-propylene glycol, lactose, sucrose, talc, particularly talc extra fine, and combinations thereof. The pore-forming agent may be present in an amount from 0.1 % to 20%, preferably from 0.1 % to 10%, by weight of the entire pellet.

The term "hydrophilic release modulator polymer" as used herein refers to a polymer that is water soluble and controls the release of the active agent. Nevertheless, in certain embodiments, the hydrophilic release modulator polymer comprised within the ER coating layer of the ER pellet of the invention acts, in fact, as a pore-forming agent. Examples of hydrophilic release modulator polymers include PVP, PEG, HPMC, HPC, and combinations thereof. The hydrophilic release modulator polymer may be present in an amount from 0.1 % to 20%, preferably from 0.1% to 10%, by weight of the entire pellet.

The term "plasticizer" as used herein includes any compound or combination of compounds capable of plasticizing or softening a polymer used in the ER pellet of the present invention. During manufacture of the ER coating layer, the plasticizer can lower the melting temperature or glass transition temperature (softening point temperature) of the polymer or combination of polymers used; can broaden the average molecular weight of said polymer or combination of polymers, and can further reduce the viscosity of said polymer or combination of polymers for convenient processing of the coat solution. Examples of plasticizers include dibutyl sebacate; dibutyl phthalate; citrate esters, such as triethylcitrate, and triacetin; propylene glycol; low molecular weight poly(alkylene oxides), such as PEG, poly(propylene glycols), and poly(ethylene/propylene glycols); and combinations thereof. The plasticizers may be present in an amount from 0.1% to 20%, preferably from 0.1% to 10%, by weight of the entire pellet.

The ER pellet of the present invention may comprise further inactive ingredients such as osmotic pressure/tonicity agent. Such agents are commonly used for time-controlled disintegration when a pulsatile drug delivery is required. Examples of suitable osmotic/tonicity excipients that may be used in the preparation of the ER pellet include sodium chloride and mannitol. The osmotic/tonicity agent when comprised in the ER pellet may be present in an amount of up to 20%, preferably from 0.5% to 10%, by weight of the entire pellet.

In particular embodiment exemplified herein, the ER pellets exemplified herein comprises an inert pellet core; a drug layer comprising the active agent admixed with PVP as a film-former polymer/binder and with talc extra fine as a glidant; and an ER coating layer comprising ethylcellulose as a pH-independent polymer, and PEG as a pore-forming agent, wherein the amount of said film-former polymer/binder is up to 90% by weight of the entire drug layer, or from 0.5% to 20% by weight of the entire pellet; the amount of said glidant is up to 30% by weight of the entire drug layer, or from 0.1% to 10% by weight of the entire pellet; the amount of said pH-independent polymer is from 50% to 90% by weight of the entire ER coating layer, or from 10% to 30% by weight of the entire pellet; and the amount of said pore-forming agent is from 1% to 20% by weight of the entire ER coating layer, or from 0.1% to 10% by weight of the entire pellet.

In other particular embodiments exemplified herein, the ER pellet of the present invention comprises an inert pellet core; a drug layer comprising said active agent admixed with PVP as a film-former polymer/binder and with talc extra fine as a glidant; an isolating/protecting sub-coating layer comprising PVP as a film-former polymer; and an ER coating layer comprising ethylcellulose as a pH-independent polymer, PEG as a pore-forming agent, and talc extra fine as a glidant, wherein the amount of said film-former polymer/binder in said drug layer is up to 90% by weight of the entire drug layer, or from 0.5% to 20% by weight of the entire pellet; the amount of said glidant in said drug layer is up to 30% by weight of the entire drug layer, or from 0.1% to 10% by weight of the entire pellet; the amount of said film-former polymer in said sub-coating layer is up to 100% by weight of the entire sub-coating layer, or from 0.5% to 20% by weight of the entire pellet; the amount of said pH-independent polymer is from 50% to 90% by weight of the entire ER coating layer, or from 10% to 30% by weight of the entire pellet; the amount of said pore-forming agent is from 1% to 20% by weight of the entire ER coating layer, or from 0.1% to 10% by weight of the entire pellet; and the amount of said glidant in said ER coating layer is from 0.1% to 20% by weight of the entire ER coating layer, or from 0.1% to 10%, by weight of the entire pellet.

In certain embodiments, the extended-release pellets are blended with one or more suitable excipients and either filled into a capsule or compressed into a tablet, wherein said capsule or tablet comprises extended-release pellets comprising extended-release pellets comprising rasagiline and extended-release pellets comprising pramipexole, or extended-release pellets comprising both rasagiline and pramipexole.

The preparation of such capsules or tablets may be carried out using any suitable technology known in the art.

Examples of suitable excipients, which may be used in the preparation of the oral pharmaceutical composition include silicon dioxides, as well as other glidants known in the art as defined above.

Tablets fillers fill out the size of a tablet or capsule, making it practical to produce and convenient for the consumer to use. By increasing the bulk volume, the fillers make it possible for the final product to have the proper volume for patient handling. A good filler must be inert, compatible with the other components of the formulation, non-hygroscopic, relatively cheap, compactible, and preferably tasteless or pleasant tasting. Plant cellulose (pure plant filler) is a popular filler in tablets or hard gelatin capsules. Dibasic calcium phosphate is another popular tablet filler. A range of vegetable fats and oils can be used in soft gelatin capsules. Tablet fillers include, e.g., lactose, mannitol/Parteck®, sorbitol, starch, and combinations thereof.

Disintegrant expand and dissolve when wet causing the tablet to break apart in the digestive tract, releasing the active ingredients for absorption. Disintegrant types include water uptake facilitators and tablet rupture promoters. They ensure that when the tablet is in contact with water, it rapidly breaks down into smaller fragments, facilitating dissolution. Examples of disintegrants include crosslinked polyvinylpyrrolidone (crospovidone), sodium/calcium carboxymethyl cellulose (CMC), croscarmellose sodium hydroxypropyl cellulose low-substiuted, sodium bicarbonate, starch, sodium starch glycolate, and combinations thereof.

Lubricants are added in small quantities to tablet and capsule formulations to improve certain processing characteristics. More particular, these agents prevent ingredients from clumping together and from sticking to the tablet punches or capsule-filling machine. Lubricants also ensure that tablet formation and ejection can occur with low friction between the solid and die wall. Examples of lubricants include glyceryl behenate, stearic acid, talc, zinc stearate, calcium stearate, and combinations thereof.

The terms "treat", "treatment" and "provide substantial therapeutic effect" are used interchangeably herein and refer to stopping, slowing down, reducing the extent of or minimizing the neurodegenerative process in nigrostriatal neurons (neuroprotective therapy), eliminating or reducing the biochemical imbalance, increasing dopamine synthesis, stimulating dopamine receptors activity and dopamine release from the presynaptic space, and/or inhibiting dopamine reuptake by presynaptic receptors and dopamine catabolism. The terms may also refer to improving or slowing worsening of symptoms of Parkinson's disease such as tremor, slowed motion (bradykinesia), Restless Leg Syndrome, rigid muscles, impaired posture and balance, loss of automatic movements, speech changes, impaired sleep and/or impaired quality of life (QOL), eliminating or reducing physical, cognitive or mental symptoms of Parkinson's disease and even possibly slowing down or arresting the progress of dementia.

Improvement, reduction or slowing of worsening of symptoms of Parkinson's disease may be measured by assessing one or more accepted parameters before and during the term of the treatment, such as the Unified Parkinson's Disease Rating Scale (UPDRS) score, UPDRS activity of daily life (ADL) and motor sub-scores, Beck Depression Inventory® - II (BDI-II), International Restless Leg Syndrome Rating Scale (IRLS) symptoms, Parkinson Disease Questionnaire 39 (PDQ39), Clinical Global Impression (CGI).

In yet another aspect, the present invention provides a method for preparing an extended release formulation of a fixed dose combination of pramipexole and rasagiline, or a pharmaceutically acceptable salt thereof, said method comprising the steps of: (i) dissolving an active agent comprising pramipexole, rasagiline or both, optionally suitably admixed with a binder and/or a glidant, in a suitable solvent system to prepare a uniform suspension; (ii) applying a coat of the suspension obtained in (i) to inert pellets such as inert nonpareil seeds; (iii) optionally coating the rasagiline loaded pellets, pramipexole-loaded pellets or pellets loaded with both rasagiline and pramipexole obtained in (ii) with an insulating/protecting sub-coating layer; (iv) coating the pellets obtained in (ii) or (iii) with an extended-release coating layer which enables an extended release of said rasagiline and pramipexole thereby obtaining said extended release formulation; (v) optionally blending the coated pellets obtained in (iv) with a suitable excipient; and (vi) filling said extended release formulation into capsules or compressing said extended release formulation into tablets, wherein said capsules or tables comprise a ratio of rasagiline loaded pellets and pramipexole-loaded pellets selected from a range of 1:1.1 to 1:20, 1:1.1 to 1:10, 1:1.1 to 1:5, 1:1.1 to 1:3 or 1:1.1 to 1:2; or said capsules or tablets comprise pellets loaded with both rasagiline and pramipexole at a ratio selected from a range of 1:1.1 to 1:20, 1:1.1 to 1:10, 1:1.1 to 1:5, 1:1.1 to 1:3 or 1:1.1 to 1:2, thereby obtaining an extended release formulation of a fixed dose combination of rasagiline and pramipexole.

The invention will now be illustrated by the following examples.

### EXAMPLES

### Example 1: Formulation and dissolution profile of the combination product.

Each component was formulated separately **(Tables 1 and 2)** and the beads were encapsulated in respective weights to give a dose of 0.6 mg pramipexole and 0.75 mg rasagiline.

### Analytical method - Dissolution Test For the combination product

The method evaluates the dissolution profile for the active pharmaceutical ingredients (API's) pramipexole (PPX) and rasagiline (RAS) in coated pellets formulated for extended release (ER), packed in capsule, by using high performance liquid chromatography (HPLC) for quantitative analysis.

The content of one capsule or one dose of beads (pellets) sample, was placed into a basket, which rotates inside a vessel containing a medium, under constant specified

**Table 1. Rasagiline mesylate ER coated pellets with sub coating**

| **Ingredients** | **Mg/capsule** (22% ER) |
|---|---|
| **Cores - drug layered coated pellets** | |
| Ethanol 96% | - |
| Distilled water | - |
| rasagiline mesylate | 1.17 |
| PVP K25 | 6.27 |
| Talc extra fine | 0.78 |
| Sugar spheres 600-710µm | 70.20 |
| Total core weight | 78.42 |

| **Cores - sub coated pellets** | |
|---|---|
| Distilled water | - |
| Ethanol 96% | - |
| PVP K25 | 2.36 |
| Total SC core weight | 80.78 |

| **Functional coating (ER coating)** | |
|---|---|
| Acetone | - |
| Ethanol 96% | - |
| Distilled water | - |
| Ethocel 45 cps | 15.99 |
| PEG 3000 | 0.89 |
| Talc extra fine | 0.89 |
| Total ER pellets weight | 98.55 |

| **Dry mix** | |
|---|---|
| Silicon dioxide colloidal | 0.09 |
| Total | 98.64 |

rate and temperature. Sample was dissolved in the medium solution over time in a rate that reflected the release profile of the formulation, thus the solution contains different concentrations of API's at different time points. Samples were taken automatically or manually at specified time points within specified time interval, filtered through 20 µm PE, Cat. No 400111, Sun Sri filter. and quantified against reference standards solutions.

The conditions used for the experiment disclosed in **Table 3** were:
- Apparatus:1 (baskets)
- Medium: Intestinal Fluid Simulated (IFS), a buffer that mimics intestinal conditions
- Speed: 100 rpm
- Temperature: 37°C ± 0.5°C

The amount of RAS and PPX dissolved was determined using HPLC.

**Table 2. Pramipexole Dihydrochloride Monohydrate ER coated pellets with sub coating.**

| **Ingredients** | **Mg/capsule** (18% ER) |
|---|---|
| **Cores - drug layered coated pellets** | |
| Ethanol 96% | - |
| Distilled water | - |
| pramipexole Dihydrochloride Monohydrate | 0.60 |
| PVP K25 | 16.54 |
| Talc extra fine | 1.90 |
| Sugar spheres 600-710µm | 104.76 |
| Total core weight | 123.80 |

| **Cores - sub coated pellets** | |
|---|---|
| Distilled water | - |
| Ethanol 96% | - |
| PVP K25 | 3.71 |
| Total SC core weight | 127.51 |

| **Functional coating (ER coating)** | |
|---|---|
| Acetone | - |
| Ethanol 96% | - |
| Distilled water | - |
| Ethocel 45 cps | 20.65 |
| PEG 3000 | 1.15 |
| Talc extra fine | 1.15 |
| Total ER pellets weight | 150.46 |

| **Dry mix** | |
|---|---|
| Silicon dioxide colloidal | 0.14 |
| Total | 150.60 |

### Results of Analytical method - Dissolution Test For the combination product capsule .

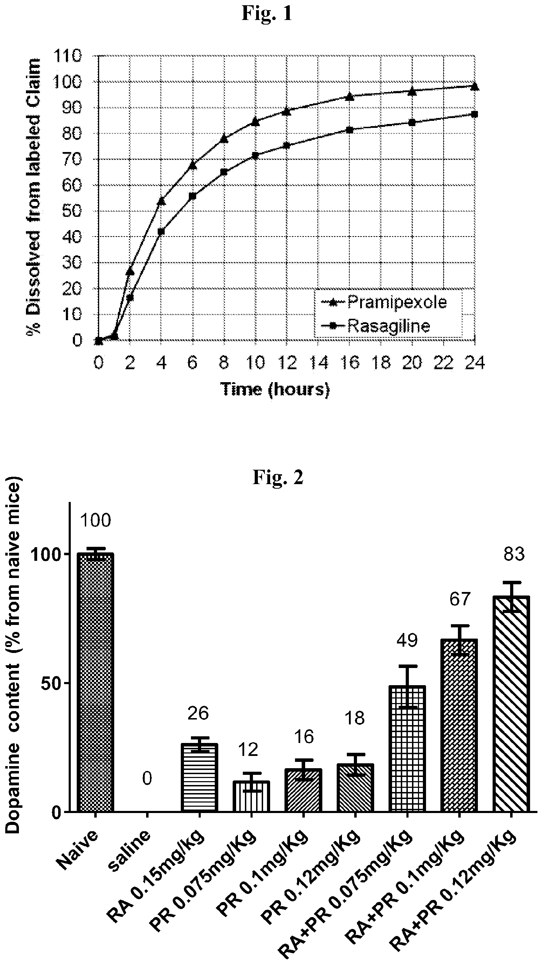

**Table 3. Dissolution profile of the combination product capsule (see Fig. 1 for graphical representation)**

| **Time (hrs)** | **Rasagiline mesylate % Dissolved** | **Pramipexole Dihydrochloride Monohydrate % Dissolved** |
|---|---|---|
| 0 | 0 | 0 |
| 1 | 1.5 | 2.3 |
| 2 | 16.5 | 27.1 |
| 4 | 42.0 | 54.1 |
| 6 | 55.8 | 68.0 |
| 8 | 65.1 | 78.3 |
| 10 | 71.7 | 84.9 |
| 12 | 75.5 | 88.8 |
| 16 | 81.5 | 94.6 |
| 20 | 84.4 | 96.6 |
| 24 | 87.6 | 98.5 |

### Example 2: In vivo study of drugs in MPTP model of Parkinson's disease.

### Material and Methods

***Models.*** Experimental models of Parkinson's disease (PD) are needed to gain insights into the possible pathological mechanisms of the disease. In addition to this function, they are essential in the development and testing of new therapeutic strategies, whether pharmacological or otherwise.

**MPTP mice model**. A significant body of biochemical data from human brain autopsy studies and those from animal models point to an ongoing process of oxidative stress in the substantia nigra which could initiate dopaminergic neurodegeneration. It is not known whether oxidative stress is a primary or secondary event. Nevertheless, oxidative stress, as induced by the neurotoxin MPTP (N-methyl-4-phenyl-1,2,3,6-tetrahydropyridine), has been used in animal models to investigate the process of neurodegeneration with the intent to develop antioxidant neuroprotective drugs.

**Table 4: Groups allocation**

| **Group (6-10 mice each group)** | **Treatments (Daily)** |
|---|---|
| 1M | Naive Saline+ Saline |
| 2M | 40 mg/kg MPTP - HCL + saline |
| 3M | 40 mg/kg MPTP - HCL + rasagiline dose 0.15mg/Kg |
| 4M | 40 mg/kg MPTP - HCL + pramipexole dose 0.12mg/Kg |
| 5M | 40 mg/kg MPTP - HCL + pramipexole dose 0.1mg/Kg |
| 6M | 40 mg/kg MPTP - HCL + pramipexole dose 0.075mg/Kg |
| 7M | 40 mg/kg MPTP - HCL + rasagiline dose 0.15mg/Kg+ pramipexole dose 0.12mg/Kg |
| 8M | 40 mg/kg MPTP - HCL + rasagiline dose 0.15mg/Kg+ pramipexole dose 0.1mg/Kg p |
| 9M | 40 mg/kg MPTP - HCL + rasagiline dose 0.15mg/Kg+ pramipexole dose 0.075mg/Kg p |

The neurotoxin MPTP is converted in the brain into the positively charged molecule MPP+ (1-methyl-4-phenylpyridinium) by the enzyme MAO-B, causing parkinsonism in primates by killing certain dopamine-producing neurons in the substantia nigra. It acts by interfering with oxidative phosphorylation in mitochondria, causing depletion of ATP and cell death. It also inhibits the synthesis of catecholamines, reduces levels of dopamine and cardiac norepinephrine, and inactivates tyrosine hydroxylase.

**Experimental procedure**: Male C57B1/6 mice weighing 20+/- 1g are used (6-10 mice per group). MPTP is administrated by intraperitonealy (IP) injection at a dose of 40mg/Kg per day for 5 days. Controls are naive untreated mice injected with saline, and MPTP treated mice injected with saline (no drug treatment). Drugs, rasagiline (0.15mg/Kg) and pramipexole (3 different doses of 0.12, 0.1 and 0.075mg/Kg), are given alone or in 3 fixed dose combinations of rasagiline and pramipexole. The fixed dose combinations are composed of rasagiline at a constant dose of 0.15 mg/Kg and pramipexole at 3 different doses as indicated above. Both drugs are dissolved together at saline from their stock solutions to give the final desired combination dose. The application of the drugs is done daily intraperitonealy (IP) injection 30 minutes before MPTP administration. Drug treatment is prolonged for 12 days. The effect of the treatments is assessed by measurement of dopamine and its metabolites (dihydroxyphenylacetic acid and Homovanillic acid) in left and right striatum together taken from the mice at the end of the experiment.

The study included 9 groups of 6-10 mice each. The mice are treated with MPTP to induce the Parkinson model, and treated with combinations with constant dose of rasagiline and varying doses of pramipexole. Controls are naïve untreated mice injected with saline, and MPTP treated mice injected with saline (no drug treatment). The groups are treated according to the **Table 4** above. The testing schedule is shown in **Table 5.**

**Table 5: Testing Time Table - First Dosing Day 0**

| Test Study Days | **Dosing (Test Compounds)** | | | | | | | | | | | | |
|---|---|---|---|---|---|---|---|---|---|---|---|---|---|
| | **Dosing (MPTP)** | | | | | | | | | | | | |
| | 0 | 1 | 2 | 3 | 4 | 5 | 6 | 7 | 8 | 9 | 10 | 11 | 12 |
| **MPTP** | v | v | v | v | v | | | | | | | | End of Study |
| **Test items** | v | v | v | v | v | v | v | v | v | v | v | v | |

**Sample preparation for HPLC analysis of Dopamine and metabolizes.** Striatum tissue samples are homogenized in ice in 500µl homogenization buffer (0.1M perchloric acid, 0.02% EDTA and 1% ETOH) using OMNI Tip homogenizing kit of OMNI International (intermediate speed, 3X 10 seconds with 5 seconds intervals). The homogenates are sonicated for 5 minutes then centrifuged at 15,000 RPM at 4°C for 15min. The supernatants are transferred into fresh tubes and Dopamine content is analyzed by HPLC.

The results of this experiment are presented in **Figure 2**. Dopamine levels in naive mice brain were normalized to 100%, while Dopamine levels of MPTP-PD-mice treated with saline were normalized to 0%. The graph reflects effect of the different treatments on Dopamine levels compared to saline. It is clearly seen that the FDC is highly synergistic, where the effect of rasagiline and all three doses of pramipexole when given as monotherapies, is very low, while the combination containing the same doses is highly effective, and notably, remarkably more effective than the sum of the effect of both components. The effect of the combination is dose dependent, with efficacy increasing in correlation with the increase in pramipexole dose, however, the dose response is remarkably more significant than the dose-response in the effect of the increasing doses of Pramipexole when given alone. This implies that the addition of Rasagiline is much more than a simple additive effect and suggest a strong synergistic effect of the combination in the reported conditions.

Thus, the mice study shows clearly, that when the drugs in the combination product of the present invention are administered as fixed dose combination, in doses that have very low or no effect as monatherapies, they give a therapeutic effect that is larger than the sum of their individual effects, indicating that this synergistic effect, probably arising from their complementary biological mechanisms. This suggests that if we employ, in a human trial, doses that are subtherapeutic, or lower than the ones currently employed as effective monotherapies, we will also see a significant effect, as can be anticipated by the results in the mice study.

### Example 3: Phase I pharmacokinetic study in healthy volunteers.

Pharma Two B conducted a 4 arms crossover study in healthy fasted volunteer adults comparing a single dose marketed immediate release rasagiline (Azilect, 1 mg), marketed extended release pramipexole (Mirapex ER, 0.75 mg), both marketed drugs taken together, and Pharma Two B's proprietary extended-release combination product containing doses of rasagiline (1 mg) and pramipexole (0.75 mg) that equal to the commercially available mono-therapy products.

**Co-Administration of Monotherapies.** Evaluation of the plasma concentrations of pramipexole showed no statistically significant effect of concomitant rasagiline administration on pramipexole pharmacokinetics. Co-administration of Mirapex ER and Azilect resulted in only a 4% decrease in pramipexole Cₘₐₓ and a 3% increase in AUC_{inf}. Similarly, no statistically significant effect of concomitant pramipexole administration was observed on rasagiline pharmacokinetics. Rasagiline Cₘₐₓ and AUC_{inf} increased 3% and 4% respectively when co-administered with Mirapex ER (**Table 6**).

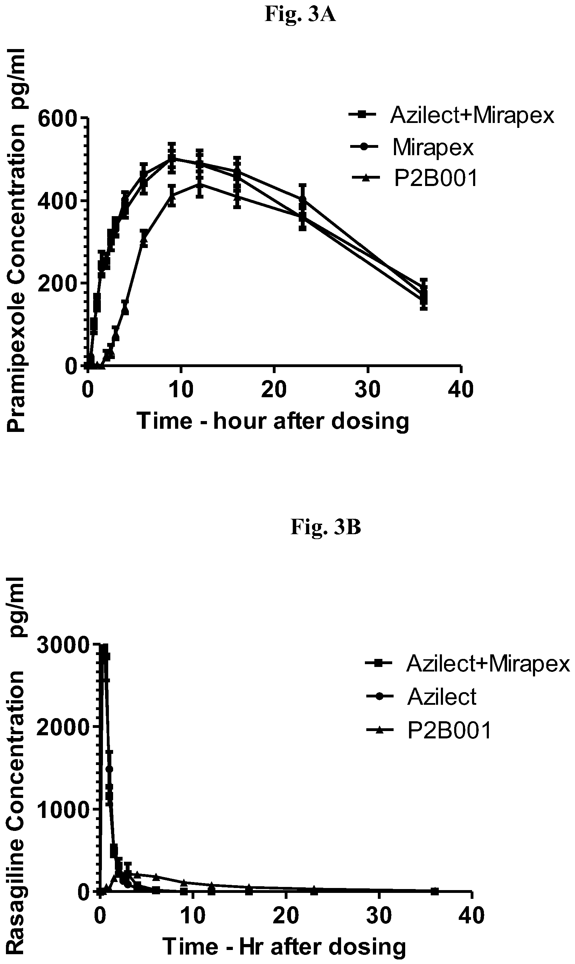

**Co-Administration of Monotherapies Versus the combination product.** The proprietary extended-release product (FDC) yielded a slightly different (statistically insignificant) pharmacokinetic profile for the pramipexole component than was observed when Mirapex ER was coadministered with Azilect (Cmax was decreased by 15% and AUC_{inf} was increased by 10% (**Table 6 and** **Figs. 3A-B**)).

**Table 6. Pharmacokinetics of pramipexole and rasagiline**

| **Treatment** | **Mean Cₘₐₓ (pg/mL)** | **Mean AUCₜ (pg·h/mL)** | **Mean AUC_{inf} (pg·h/mL)** | **Mean Tₘₐₓ (h)** |
|---|---|---|---|---|
| **pramipexole** | | | | |
| Mirapex ER | 573.8 | 13427.5 | 15889.4 | 11.7 |
| Mirapex ER+Azilect | 550.6 | 12671.1 | 16285.3 | 11.2 |
| Pharma 2B combination product | 469.6 | 10937.7 | 17910.7 | 13.4 |

| **rasagiline** | | | | |
|---|---|---|---|---|
| Azilect | 4808.1 | 3475.2 | 3533.4 | 0.5 |
| Azilect +Mirapex ER | 4928.5 | 3648.1 | 3678.5 | 0.6 |
| Pharma 2B combination product | 275.9 | 2244.9 | 2774.8 | 3.4 |

The pharmacokinetic profile of the rasagiline component of the combination product reflected the differences in formulation (extended release versus immediate release) used in the other study arms. The Cₘₐₓ of rasagiline from the combination product was approximately 95% lower while the AUC_{inf} was 22% lower than that of Azilect when coadministered with Mirapex ER.

Taken together, this study shows that co-administration of pramipexole and rasagiline has no effect on the pharmacokinetic profiles of the two drugs and that the combination product formulation delivers pramipexole similar to Mirapex ER. Also, the combination product formulation yields an extended-release of rasagiline without significantly impacting the overall exposure to the drug. Furthermore, these data suggest a promising safety profile of drug combination.

### Example 4. Phase IIB clinical trial for evaluation of Applicant's fixed dose combination product.

Applicant intends to show that their proprietary combination product has benefits and is well-tolerated with a good safety profile in early Parkinson's disease patients.

This is done by utilizing a dose-ranging study comparing 3 doses of Pharma 2b combination product to placebo in order to study the safety, tolerability and efficacy of this therapy and to identify the best low dose combination that will lead to the highest clinical efficacy concurrently with reduced side effects.

The primary objective is the assessment of efficacy, safety and tolerability of 3 different doses of the combination product and the secondary objective is the assessment of the effect of the combination product on sleep, mood and quality of life (QOL).

**Study plan.** Applicant's study is a phase IIB, randomized, double blind, placebo-controlled, parallel groups, multi-centre, dose-ranging study with 3 combination doses. Each component dose is used at a lower dose than is commonly used in the management of patients with early stage Parkinson's disease. The study population includes 200 early stage Parkinson's disease patient volunteers (Parkinson's disease diagnosis consistent with UK Parkinson's Disease Society Brain Bank Clinical Diagnostic Criteria and Modified Hoehn and Yahr staging < 3), that are recruited from community and academic hospitals in USA and Israel at a total of 45 sites, among them about 40 sites in USA and 5 in Israel.

Patient volunteers are randomly assigned to one of four treatment groups (50 subjects per group): and receive one of three different doses of the combination product, where the doses of rasagiline will vary between 0.1mg to 0.75 mg and the dose of pramipexole will vary from 0.1 to 0.6 mg, or a matching placebo.

All treatments are taken orally once a day in the morning before breakfast, at about the same time every day.

The study is divided into 3 phases; screening phase (maximum of 4 weeks), treatment and maintenance phase (12 weeks) and additional safety phase (2 weeks).

The following outcomes are measured: Efficacy in which the primary endpoint is the change from baseline to final visit (week 12) in total Unified Parkinson's Disease Rating Scale (UPDRS) score (defined as sum of parts II -activity of daily life and III-motor evaluation, scores between 0 to 160). Secondary endpoints will include change from baseline to 12 weeks in UPDRS activity of daily life (ADL) and motor sub-scores, Beck Depression Inventory® - II (BDI-II), Parkinson Disease Questionnaire 39 (PDQ39), and Clinical Global Impression (CGI) of subject and investigator- Safety will be assessed by adverse event reporting (frequency and incidence) and scale assessments of sleep, daytime sleepiness, depression, suicidality, impulse control behaviors. Tolerability will be assessed by percentage of subjects that complete the trial on treatment assignment. Over the 12-week trial UPDRS assessments and clinical safety evaluations will be made every 2-4 weeks. The primary analysis of efficacy will compare active dosage groups to placebo using a mixed model repeated measures (MMRM).

## Claims

1. A pharmaceutical composition for use in the treatment of Parkinson's disease comprising a pharmaceutically acceptable carrier and a fixed dose combination of pramipexole and rasagiline, wherein the fixed dose combination contains from 0.05 mg to 1.0 mg of pramipexole and from 0.05 mg to 1.0 mg of rasagiline, and the dose of pramipexole is lower than the dose of rasagiline.

2. The pharmaceutical composition for use according to claim 1, wherein the molar ratio of pramipexole to rasagiline is selected from a range of 1:1.1 to 1:20, 1:1.1 to 1:10, 1:1.1 to 1:5,1:1.1 to 1:3 or 1:1.1 to 1:2.

3. The pharmaceutical composition for use according to claim 1 or 2, wherein the fixed dose combination contains from 0.1 mg to 0.6 mg pramipexole and from 0.1 mg to 0.75 mg rasagiline.

4. The pharmaceutical composition for use according to any one of claims 1 to 3, wherein said pramipexole and rasagiline are formulated for extended release.

5. The pharmaceutical composition for use according to claim 4, in the form of a monolithic matrix; a tablet, preferably a bi- or multi-layered tablet, matrix tablet, disintegrating tablet, dissolving tablet, or chewable tablet; a capsule or sachet, preferably filled with granules, grains, beads, or pellets; or a depot system based on a biodegradable polymer such as poly(D,L-lactide) (PLA), polyglycolide (PGA), and poly(D,L-lactide-co-glycolide) (PLGA).

6. The pharmaceutical composition for use according to any one of claims 1-5, formulated for oral administration.

7. The pharmaceutical composition for use according to claim 6 comprising extended-release pellets comprising:
(i) an inert pellet core;
(ii) a drug layer coating said pellet core, said drug layer comprising an active agent comprising pramipexole, rasagiline or both, or a pharmaceutically acceptable salt thereof, optionally suitably admixed with a binder and/or a film-former polymer, and further optionally admixed with a glidant;
(iii) optionally an isolating/protecting sub-coating layer coating said drug layer; and
(iv) an extended-release coating layer coating said sub-coating layer, if present, or said drug layer.

8. The pharmaceutical composition for use according to claim 7, wherein said sub-coating layer comprises a film-former polymer and optionally a glidant.

9. The pharmaceutical composition for use according to claim 8 , wherein said extended-release coating layer comprises:
(i) at least one pH-independent polymer and optionally a pore-forming agent, wherein the extended-release pellet has a pH-independent *in vitro* release characteristic;
(ii) a pH-independent polymer, a hydrophilic release modulator polymer, and optionally a hydrophobic or hydrophilic plasticizer, and/or a glidant; or
(iii) a mixture of a pH-dependent enteric-coating polymer and a pH-independent polymer, wherein the extended-release pellet has a close to zero order *in vitro* release characteristic at pH value of up to pH 7.4.

10. The pharmaceutical composition for use according to any one of claims 7 to 9, wherein:
(i) said binder is a polyvinyl pyrrolidone (PVP), hydroxypropylmethyl cellulose (HPMC), hydroxypropyl cellulose (HPC), microcrystalline cellulose, or a combination thereof;
(ii) said film-former polymer is PVP, HPMC, HPC, microcrystalline cellulose, or a combination thereof;
(iii) said glidant is talc, colloidal silicon dioxide, glyceryl monostearate, or a combination thereof;
(iv) said pH-independent polymer is ethyl cellulose, a copolymer of ethylacrylate, methylmethacrylate and trimethylammonioethyl methacrylate chloride, 1:2:0.2; a copolymer of ethylacrylate, methyl methacrylate and trimethylammonioethyl methacrylate chloride, 1:2:0.1; a copolymer of ethylacrylate and methylmethacrylate), 2:1; or a combination thereof;
(v) said pH-dependent enteric-coating polymer is a copolymer of (methacrylicacid and methylmethacrylate, 1:2; a copolymer of methacrylicacid and ethylacrylate, 1:1; polyvinyl alcohol-polyethylene glycol graft copolymer; hydroxypropylmethyl cellulose phthalate (HPMCP); alginates; carboxymethylcellulose; or a combination thereof;
(vi) said pore-forming agent is PVP, PEG, HPMC, HPC, methylcellulose, 1,2-propylene glycol, lactose, sucrose, talc, or a combination thereof;
(vii) said hydrophilic release modulator polymer is HPMC, HPC, PVP, PEG, or a combination thereof; and
(viii) said plasticizer is dibutyl sebacate; dibutyl phthalate; citrate esters such as triethylcitrate and triacetin; propylene glycol; poly(alkylene oxides) such as PEG, poly(propylene glycols), and poly(ethylenelpropylene glycols); or a combination thereof.

11. The pharmaceutical composition for use according to any one of claims 7 to 10, wherein said extended release pellet comprises:
(i) an inert pellet core; a drug layer comprising said active agent admixed with PVP as a film-former polymer/binder and with talc extra fine as a glidant; and an extended-release (ER) coating layer comprising ethylcellulose as a pH-independent polymer, and PEG as a pore-forming agent, wherein the amount of said film-former polymer/binder is up to 90% by weight of the entire drug layer, or from 0.5% to 20% by weight of the entire pellet; the amount of said glidant is up to 30% by weight of the entire drug layer, or from 0.1% to 10% by weight of the entire pellet; the amount of said pH-independent polymer is from 50% to 90% by weight of the entire ER coating layer, or from 10% to 30% by weight of the entire pellet; and the amount of said pore-forming agent is from 1% to 20% by weight of the entire ER coating layer, or from 0.1% to 10% by weight of the entire pellet; or
(ii) an inert pellet core; a drug layer comprising said active agent admixed with PVP as a film-former polymer/binder and with talc extra fine as a glidant; an isolating/protecting sub-coating layer comprising PVP as a film-former polymer; and an ER coating layer comprising ethylcellulose as a pH-independent polymer, PEG as a pore-forming agent, and talc extra fine as a glidant, wherein the amount of said film-former polymer/binder in said drug layer is up to 90% by weight of the entire drug layer, or from 0.5% to 20% by weight of the entire pellet; the amount of said glidant in said drug layer is up to 30% by weight of the entire drug layer, or from 0.1% to 10% by weight of the entire pellet; the amount of said film-former polymer in said sub-coating layer is up to 100% by weight of the entire sub-coating layer, or from 0.5% to 20% by weight of the entire pellet; the amount of said pH-independent polymer is from 50% to 90% by weight of the entire ER coating layer, or from 10% to 30% by weight of the entire pellet; the amount of said pore-forming agent is from 1% to 20% by weight of the entire ER coating layer, or from 0.1% to 10% by weight of the entire pellet; and the amount of said glidant in said ER coating layer is from 0.1% to 20% by weight of the entire ER coating layer, or from 0.1% to 10%, by weight of the entire pellet.

12. The pharmaceutical composition for use according to any one of claims 7 to 11, wherein said extended-release pellets are blended with one or more suitable excipients and either filled into a capsule or compressed into a tablet, and wherein said capsule or tablet comprises extended-release pellets comprising pramipexole and extended-release pellets comprising rasagiline, or extended-release pellets comprising both pramipexole and rasagiline.

13. A method for preparing an extended release formulation of a fixed dose combination of pramipexole and rasagiline, or a pharmaceutically acceptable salt thereof, said method comprising the steps of:
(i) dissolving an active agent comprising pramipexole, rasagiline or both, optionally suitably admixed with a binder and/or a glidant, in a suitable solvent system to prepare a uniform suspension;
(ii) applying a coat of the suspension obtained in (i) to inert pellets such as inert nonpareil seeds;
(iii) optionally coating the rasagiline loaded pellets, pramipexole-loaded pellets or pellets loaded with both pramipexole and rasagiline, obtained in (ii) with an insulating/protecting sub-coating layer;
(iv) coating the pellets obtained in (ii) or (iii) with an extended-release coating layer which enables an extended release of said pramipexole and rasagiline thereby obtaining said extended release formulation;
(v) optionally blending the coated pellets obtained in (iv) with a suitable excipient; and
(vi) filling said extended release formulation into capsules or compressing said extended release formulation into tablets, wherein said capsules or tablets comprise a ratio of pramipexole-loaded pellets and rasagiline-loaded pellets selected from a range of 1:1.1 to 1:20, 1:1.1 to 1:10, 1:1.1 to 1:5, 1:1.1 to 1:3 or 1:1.1 to 1:2; or said capsules or tablets comprise pellets loaded with both pramipexole and rasagiline at a ratio selected from a range of 1:1.1 to 1:20, 1:1.1 to 1:10,1:1.1 to 1:5, 1:1.1 to 1:3 or 1:1.1 to 1:2,
thereby obtaining an extended release formulation of a fixed dose combination of pramipexole and rasagiline.

## Patentansprüche

1. Pharmazeutische Zusammensetzung zur Verwendung bei der Behandlung von Parkinson-Krankheit, umfassend einen pharmazeutisch verträglichen Träger und eine Festdosis-Kombination aus Pramipexol und Rasagilin, wobei die Festdosis-Kombination von 0,05 mg bis 1,0 mg Pramipexol und von 0,05 mg bis 1,0 mg Rasagilin enthält und die Dosierung von Pramipexol niedriger als die Dosierung von Rasagilin ist.

2. Pharmazeutische Zusammensetzung zur Verwendung nach Anspruch 1, wobei das Molverhältnis von Pramipexol zu Rasagilin in einem Bereich liegt, der ausgewählt ist aus 1:1,1 bis 1:20, 1:1,1 bis 1:10, 1:1,1 bis 1:5, 1:1,1 bis 1:3 oder 1:1,1 bis 1:2.

3. Pharmazeutische Zusammensetzung zur Verwendung nach Anspruch 1 oder 2, wobei die Festdosis-Kombination von 0,1 mg bis 0,6 mg Pramipexol und von 0,1 mg bis 0,75 mg Rasagilin enthält.

4. Pharmazeutische Zusammensetzung zur Verwendung nach einem der Ansprüche 1 bis 3, wobei das Pramipexol und das Rasagilin für die verlängerte Freisetzung formuliert sind.

5. Pharmazeutische Zusammensetzung zur Verwendung nach Anspruch 4 in Form einer monolithischen Matrix; einer Tablette, vorzugsweise eine zwei- oder mehrschichtigen Tablette, einer Matrixtablette, einer zerfallenden Tablette, einer sich auflösenden Tablette oder Kautablette; einer Kapsel oder eines Beutels, vorzugsweise mit Granalien, Körnern, Perlen oder Pellets gefüllt; oder eines Depotsystems auf der Basis eines biologisch abbaubaren Polymers wie Poly(D,L-Lactid) (PLA), Polyglycolid (PGA) und Poly(D,L-Lactid-Coglycolid) (PLGA).

6. Pharmazeutische Zusammensetzung zur Verwendung nach einem der Ansprüche 1 bis 5, formuliert als orale Darreichungsform.

7. Pharmazeutische Zusammensetzung zur Verwendung nach Anspruch 6, umfassend Pellets mit verlängerter Freisetzung, umfassend:
(i) einen inerten Pelletkern;
(ii) eine Arzneimittelschicht, die den Pelletkern beschichtet, wobei die Arzneimittelschicht einen Wirkstoff umfasst, der Pramipexol, Rasagilin oder beides umfasst, oder ein pharmazeutisch verträgliches Salz davon, das wahlweise in geeigneter Weise mit einem Bindemittel und/oder einem Filmbildnerpolymer vermischt ist, und ferner wahlweise mit einem Gleitmittel vermischt ist;
(iii) wahlweise eine isolierende/schützende Teilbeschichtungsschicht, welche die Arzneimittelschicht beschichtet; und
(iv) eine Überzugsschicht mit verlängerter Freisetzung, welche die Teilbeschichtungsschicht, falls vorhanden, oder die Arzneimittelschicht beschichtet.

8. Pharmazeutische Zusammensetzung zur Verwendung nach Anspruch 7, wobei die Teilbeschichtungsschicht ein Filmbildnerpolymer und wahlweise ein Gleitmittel umfasst.

9. Pharmazeutische Zusammensetzung zur Verwendung nach Anspruch 8, wobei die Überzugsschicht mit verlängerter Freisetzung umfasst:
(i) mindestens ein pH-unabhängiges Polymer und wahlweise einen Porenbildner, wobei das Pellet mit verlängerter Freisetzung ein pH-unabhängiges in vitro-Freisetzungsmerkmal aufweist;
(ii) ein pH-unabhängiges Polymer, ein hydrophiles Freisetzungsmodulatorpolymer und wahlweise einen hydrophoben oder hydrophilen Weichmacher und/oder ein Gleitmittel; oder
(iii) eine Mischung aus einem pH-abhängigen magensaftresistenten Polymer und einem pH-unabhängigen Polymer, wobei das Pellet mit verlängerter Freisetzung ein in vitro-Freisetzungsmerkmal in einer Größenordnung von nahezu null bei einem pH-Wert von bis zu 7,4 aufweist.

10. Pharmazeutische Zusammensetzung nach einem der Ansprüche 7 bis 9, wobei:
(i) das Bindemittel ein Polyvinylpyrrolidon (PVP), Hydroxypropylmethylcellulose (HPMC), Hydroxypropylcellulose (HPC), mikrokristalline Cellulose oder eine Kombination davon ist;
(ii) das Filmbildnerpolymer PVP, HPMC, HPC, mikrokristalline Cellulose oder eine Kombination davon ist;
(iii) das Gleitmittel Talk, kolloidales Siliciumdioxid, Glycerylmonostearat oder eine Kombination davon ist;
(iv) das pH-unabhängige Polymer Ethylcellulose, ein Copolymer aus Ethylacrylat, Methylmethacrylat und Trimethylammonioethylmethacrylatchlorid, 1:2:0,2; ein Copolymer aus Ethylacrylat, Methylmethacrylat und Trimethylammonioethylmethacrylatchlorid, 1:2:0,1; ein Copolymer aus Ethylacrylat und Methylmethacrylat, 2:1; oder eine Kombination davon ist;
(v) das pH-abhängige magensaftresistente Polymer ein Copolymer aus Methacrylsäure und Methylmethacrylat, 1:2, ein Copolymer aus Methacrylsäure und Ethylacrylat, 1:1, ein Polyvinylalkohol-Polyethylenglykol-Propfcopolymer, ein Hydroxypropylmethylcellulosephthalat (HPMCP); Alginate, Carboxymethylcellulose oder eine Kombination davon ist;
(vi) der Porenbildner PVP, PEG, HPMC, HPC, Methylcellulose, 1,2-Propylenglycol, Lactose, Saccharose, Talk oder eine Kombination davon ist;
(vii) das hydrophile Freisetzungsmodulatorpolymer HPMC, HPC, PVP, PEG oder eine Kombination davon ist; und
(viii) der Weichmacher Dibutylsebacat; Dibutylphthalat; Citratester wie Triethylcitrat und Triacetin; Propylenglycol; Poly(alkylenoxid) wie PEG, Poly(propylenglycole) und Poly(ethylen/propylenglycole); oder eine Kombination davon ist.

11. Pharmazeutische Zusammensetzung zur Verwendung nach einem der Ansprüche 7 bis 10, wobei das Pellet mit verlängerter Freisetzung umfasst:
(i) einen inerten Pelletkern; eine Arzneimittelschicht, umfassend den mit PVP gemischten Wirkstoff als Filmbildnerpolymer/Bindemittel und mit extrafeinem Talk als Gleitmittel; und eine Überzugsschicht mit verlängerter Freisetzung (ER), die Ethylcellulose als ein pH-unabhängiges Polymer umfasst, und PEG als einen Porenbildner, wobei die Menge des Filmbildnerpolymers/Bindemittels bis zu 90 Ges.-% der gesamten Arzneimittelschicht oder von 0,5 bis 20 Gew.-% des gesamten Pellets beträgt; die Menge des Gleitmittels bis zu 30 Gew.-% der gesamten Arzneimittelschicht oder von 0,1 bis 10 Gew.-% des gesamten Pellets beträgt; die Menge des pH-unabhängigen Polymers von 50 bis 90 Ges.-% der gesamten ER-Überzugsschicht oder von 10 bis 30 Ges.-% des gesamten Pellets beträgt; und die Menge des Porenbildners von 1 bis 20 Gew.-% der gesamten ER-Überzugsschicht oder von 0,1 bis 10 Gew.-% des gesamten Pellets beträgt; oder
(ii) einen inerten Pelletkern; eine Arzneimittelschicht, umfassend den mit PVP gemischten Wirkstoff als Filmbildnerpolymer/Bindemittel und mit extrafeinem Talk als Gleitmittel; eine isolierende/schützende Teilbeschichtungsschicht, umfassend PVP als Filmbildnerpolymer; und eine ER-Überzugsschicht, die Ethylcellulose als ein pH-unabhängiges Polymer umfasst, und PEG als einen Porenbildner, und extrafeinen Talk als Gleitmittel, wobei die Menge des Filmbildnerpolymers/Bindemittels in der Arzneimittelschicht bis zu 90 Ges.-% der gesamten Arzneimittelschicht oder von 0,5 bis 20 Gew.-% des gesamten Pellets beträgt; die Menge des Gleitmittels in der Arzneimittelschicht bis zu 30 Ges.-% der gesamten Arzneimittelschicht oder von 0,1 bis 10 Gew.-% des gesamten Pellets beträgt; die Menge des Filmbildnerpolymers in der Teilbeschichtungsschicht bis zu 100 Gew.-% der gesamten Teilbeschichtungsschicht oder von 0,5 bis 20 Gew.-% des gesamten Pellets beträgt; die Menge des pH-unabhängigen Polymers von 50 bis 90 Gew.-% der gesamten ER-Überzugsschicht oder von 10 bis 30 Gew.-% des gesamten Pellets beträgt; die Menge des Porenbildners von 1 bis 20 Gew.-% der gesamten ER-Überzugsschicht oder von 0,1 bis 10 Gew.-% des gesamten Pellets beträgt; und die Menge des Gleitmittels in der ER-Überzugsschicht von 0,1 bis 20 Gew.-% der gesamten ER-Überzugsschicht oder von 0,1 bis 10 Gew.-% des gesamten Pellets beträgt.

12. Pharmazeutische Zusammensetzung zur Verwendung nach einem der Ansprüche 7 bis 11,
wobei die Pellets mit verlängerter Freisetzung mit einem oder mehreren geeigneten Hilfsstoffen vermischt werden und entweder in eine Kapsel gefüllt oder zu einer Tablette verpresst werden, und wobei die Kapsel oder Tablette Pellets mit verlängerter Freisetzung umfassen, die Pramipexol umfassen, und Pellets mit verlängerter Freisetzung, die Rasagilin umfassen, oder Pellets mit verlängerter Freisetzung, die sowohl Pramipexol als auch Rasagilin umfassen.

13. Verfahren zur Herstellung einer Formulierung mit verlängerter Freisetzung einer Festdosis-Kombination aus Pramipexol und Rasagilin oder eines pharmazeutisch verträglichen Salzes davon, wobei das Verfahren die folgenden Schritte umfasst:
(i) Auflösen eines Wirkstoffs, der Pramipexol, Rasagilin oder beide umfasst,
die wahlweise in geeigneter Weise mit einem Bindemittel und/oder einem Gleitmittel in einem geeigneten Lösungsmittelsystem vermischt werden, um eine gleichförmige Suspension herzustellen;
(ii) Auftragen einer Beschichtung der in (i) erhaltenen Suspension auf inerte Pellets wie inerte Nonpareilsamen;
(iii) wahlweise Beschichten der mit Rasagilin geladenen Pellets, mit Pramipexol geladenen Pellets oder Pellets, die sowohl mit Pramipexol als auch mit Rasagilin geladen sind, die in (ii) erhalten wurden, mit einer isolierenden/schützenden Teilbeschichtungsschicht;
(iv) Beschichten der in (ii) oder (iii) erhaltenen Pellets mit einer Überzugsschicht mit verlängerter Freisetzung, die eine verlängerte Freisetzung des Pramipexols und Rasagilins ermöglicht, dadurch Erhalten der Formulierung mit verlängerter Freisetzung;
(v) wahlweise Vermischen der in (iv) erhaltenen beschichteten Pellets mit einem geeigneten Hilfsstoff; und
(vi) Füllen der Formulierung mit verlängerter Freisetzung in Kapseln oder Verpressen der Formulierung mit verlängerter Freisetzung zu Tabletten, wobei die Kapseln oder Tabletten ein Verhältnis von mit Pramipexol beladenen Pellets und mit Rasagilin beladenen Pellets umfassen, das ausgewählt ist aus einem Bereich von 1:1,1 bis 1:20, 1:1,1 bis 1:10, 1:1,1 bis 1:5, 1:1,1 bis 1:3 oder 1:1,1 bis 1:2; oder die Kapseln oder Tabletten Pellets umfassen, die sowohl mit Pramipexol als auch mit Rasagilin beladen sind, in einem Verhältnis, das ausgewählt ist aus einem Bereich von 1:1,1 bis 1:20, 1:1,1 bis 1:10, 1:1,1 bis 1:5, 1:1,1 bis 1:3 oder 1:1,1 bis 1:2,
dadurch Erhalten einer Formulierung mit verlängerter Freisetzung einer Festdosis-Kombination aus Pramipexol und Rasagilin.

## Revendications

1. Composition pharmaceutique pour utilisation dans le traitement de la maladie de Parkinson comprenant un véhicule pharmaceutiquement acceptable et une combinaison de doses fixes de pramipexole et de rasagiline, dans laquelle la combinaison de doses fixes contient de 0,05 mg à 1,0 mg de pramipexole et de 0,05 mg à 1,0 mg de rasagiline, et la dose de pramipexole est inférieure à la dose de rasagiline.

2. Composition pharmaceutique pour utilisation selon la revendication 1, dans laquelle le rapport molaire pramipexole à rasagiline est choisi dans l'intervalle de 1 : 1,1 à 1 : 20, de 1 : 1,1 à 1 : 10, de 1 : 1,1 à 1 : 5, de 1 : 1,1 à 1 : 3 ou de 1 : 1,1 à 1 : 2.

3. Composition pharmaceutique pour utilisation selon la revendication 1 ou 2, dans laquelle la combinaison de doses fixes contient de 0,1 mg à 0,6 mg de pramipexole et de 0,1 mg à 0,75 mg de rasagiline.

4. Composition pharmaceutique pour utilisation selon l'une quelconque des revendications 1 à 3, dans laquelle ledit pramipexole et ladite rasagiline sont formulés pour une libération prolongée.

5. Composition pharmaceutique pour utilisation selon la revendication 4, sous la forme d'une matrice monolithique ; d'un comprimé, de préférence d'un comprimé bi- ou multi-couches, d'un comprimé matriciel, d'un comprimé à délitement, d'un comprimé à dissoudre, ou d'un comprimé à mâcher ; d'une capsule ou d'un sachet, de préférence rempli avec des granules, des grains, des billes ou des pastilles ; ou d'un système de dépôt à base d'un polymère biodégradable tel que le poly(D,L-lactide) (PLA), le polyglycolide (PGA), et le poly(D,L-lactide-co-glycolide) (PLGA).

6. Composition pharmaceutique pour utilisation selon l'une quelconque des revendications 1 à 5, formulée pour une administration orale.

7. Composition pharmaceutique pour utilisation selon la revendication 6 comprenant des pastilles à libération prolongée comprenant :
(i) un coeur inerte de pastille ;
(ii) une couche de médicament revêtant ledit coeur de pastille, ladite couche de médicament comprenant un agent actif comprenant du pramipexole, de la rasagiline ou les deux, ou un sel pharmaceutiquement acceptable de ceux-ci, optionnellement mélangé de manière appropriée avec un liant et/ou un polymère filmogène, et en outre optionnellement mélangé avec un agent de glissement ;
(iii) optionnellement une sous-couche de revêtement d'isolation/protection revêtant ladite couche de médicament, et
(iv) une couche de revêtement à libération prolongée revêtant ladite sous-couche de revêtement, si elle est présente, ou ladite couche de médicament.

8. Composition pharmaceutique pour utilisation selon la revendication 7, dans laquelle ladite sous-couche de revêtement comprend un polymère filmogène et optionnellement un agent de glissement.

9. Composition pharmaceutique pour utilisation selon la revendication 8, dans laquelle ladite couche de revêtement à libération prolongée comprend :
(i) au moins un polymère non dépendant du pH et optionnellement un agent porogène, dans lequel la pastille à libération prolongée a une caractéristique de libération *in vitro* non dépendante du pH ;
(ii) un polymère non dépendant du pH, un polymère hydrophile modulateur de libération, et optionnellement un plastifiant hydrophobe ou hydrophile, et/ou un agent de glissement ; ou
(iii) un mélange contenant un polymère de revêtement entérique dépendant du pH et un polymère non dépendant du pH, dans lequel la pastille à libération prolongée a une caractéristique de libération *in vitro* d'ordre proche de zéro à une valeur de pH allant jusqu'à pH 7,4.

10. Composition pharmaceutique pour utilisation selon l'une quelconque des revendications 7 à 9, dans laquelle :
(i) ledit liant est une polyvinylpyrrolidone (PVP), une hydroxypropylméthylcellulose (HPMC), une hydroxypropylcellulose (HPC), une cellulose microcristalline, ou une combinaison de celles-ci ;
(ii) ledit polymère filmogène est de la PVP, une HPMC, un HPC, une cellulose microcristalline, ou une combinaison de celles-ci ;
(iii) ledit agent de glissement est le talc, le dioxyde de silicium colloïdal, le monostéarate de glycéryle, ou une combinaison de ceux-ci ;
(iv) ledit polymère non dépendant du pH est l'éthylcellulose, un copolymère d'acrylate d'éthyle, de méthacrylate de méthyle et de chlorure de méthacrylate de triméthylammonioéthyle, 1 : 2 : 0,2 ; un copolymère d'acrylate d'éthyle, de méthacrylate de méthyle et de chlorure de méthacrylate de triméthylammonioéthyle, 1 : 2 : 0,1 ; un copolymère d'acrylate d'éthyle et de méthacrylate de méthyle, 2 : 1 ; ou une combinaison de ceux-ci ;
(v) ledit polymère de revêtement entérique dépendant du pH est un copolymère d'acide méthacrylique et de méthacrylate de méthyle, 1 : 2 ; un copolymère d'acide méthacrylique et d'acrylate d'éthyle, 1 : 1 ; un copolymère greffé de polyalcool vinylique-polyéthylène glycol ; le phtalate d'hydroxypropylméthylcellulose (HPMCP) ; des alginates ; une carboxyméthylcellulose ; ou une combinaison de ceux-ci ;
(vi) ledit agent porogène est une PVP, un PEG, une HPMC, une HPC, une méthylcellulose, le 1,2-propylène glycol, le lactose, le saccharose, le talc, ou une combinaison de ceux-ci ;
(vii) ledit polymère hydrophile modulateur de libération est une HPMC, une HPC, une PVP, un PEG, ou une combinaison de ceux-ci ; et
(viii) ledit plastifiant est le sébacate de dibutyle ; le phtalate de dibutyle ; des esters de citrate tels que le citrate de triéthyle et la triacétine ; le propylène glycol ; des poly(oxydes d'alkylène) tels qu'un PEG, des poly(propylène glycols), et des poly(éthylène/propylène glycols) ; ou une combinaison de ceux-ci.

11. Composition pharmaceutique pour utilisation selon l'une quelconque des revendications 7 à 10, dans laquelle ladite pastille à libération prolongée comprend :
(i) un coeur inerte de pastille ; une couche de médicament comprenant ledit agent actif mélangé avec une PVP en tant que polymère filmogène/liant et avec du talc extrafin en tant qu'agent de glissement ; et une couche de revêtement à libération prolongée (ER) comprenant une éthylcellulose en tant que polymère non dépendant du pH, et un PEG en tant qu'agent porogène, dans laquelle la quantité dudit polymère filmogène/liant est de jusqu'à 90 % en poids de la totalité de la couche de médicament, ou de 0,5 % à 20 % en poids de la pastille entière ; la quantité dudit agent de glissement est de jusqu'à 30 % en poids de la totalité de la couche de médicament, ou de 0,1 % à 10 % en poids de la pastille entière ; la quantité dudit polymère non dépendant du pH est de 50 % à 90 % en poids de toute la couche de revêtement ER, ou de 10 % à 30 % en poids de la pastille entière ; et la quantité dudit agent porogène est de 1 % à 20 % en poids de la totalité de la couche de revêtement ER, ou de 0,1 % à 10 % en poids de la pastille entière ; ou
(ii) un coeur inerte de pastille ; une couche de médicament comprenant ledit agent actif mélangé avec une PVP en tant que polymère filmogène/liant et avec du talc extrafin en tant qu'agent de glissement ; une sous-couche de revêtement d'isolation/protection comprenant une PVP en tant que polymère filmogène ; et une couche de revêtement ER comprenant une éthylcellulose en tant que polymère non dépendant du pH, un PEG en tant qu'agent porogène, et du talc extrafin en tant qu'agent de glissement, dans laquelle la quantité dudit polymère filmogène/liant dans ladite couche de médicament est de jusqu'à 90 % en poids de la totalité de la couche de médicament, ou de 0,5 % à 20 % en poids de la pastille entière ; la quantité dudit agent de glissement dans ladite couche de médicament est de jusqu'à 30 % en poids de la totalité de la couche de médicament, ou de 0,1 % à 10 % en poids de la pastille entière ; la quantité dudit polymère filmogène dans ladite sous-couche de revêtement est de jusqu'à 100 % en poids de la totalité de la sous-couche de revêtement, ou de 0,5 % à 20 % en poids de la pastille entière ; la quantité dudit polymère non dépendant du pH est de 50 % à 90 % en poids de toute la couche de revêtement ER, ou de 10 % à 30 % en poids de la pastille entière ; la quantité dudit agent porogène est de 1 % à 20 % en poids de la totalité de la couche de revêtement ER, ou de 0,1 % à 10 % en poids de la pastille entière ; et la quantité dudit agent de glissement dans ladite couche de revêtement ER est de 0,1 % à 20 % en poids de toute couche de revêtement ER, ou de 0,1 % à 10 % en poids de la pastille entière.

12. Composition pharmaceutique pour utilisation selon l'une quelconque des revendications 7 à 11, dans laquelle lesdites pastilles à libération prolongée sont mélangées avec un ou plusieurs excipients appropriés et sont soit introduites dans une capsule, soit comprimées en comprimé, et dans laquelle ladite capsule ou ledit comprimé comprend des pastilles à libération prolongée comprenant du pramipexole et des pastilles à libération prolongée comprenant de la rasagiline, ou des pastilles à libération prolongée comprenant à la fois du pramipexole et de la rasagiline.

13. Procédé de préparation d'une formulation à libération prolongée d'une combinaison de doses fixes de pramipexole et de rasagiline, ou d'un sel pharmaceutiquement acceptable de ceux-ci, ledit procédé comprenant les étapes suivantes :
(i) la dissolution d'un agent actif comprenant du pramipexole, de la rasagiline ou les deux, optionnellement mélangé de manière appropriée avec un liant et/ou un agent de glissement, dans un système de solvants approprié pour préparer une suspension uniforme ;
(ii) l'application d'un revêtement de la suspension obtenue en (i) sur des pastilles inertes telles que des grains nonpareilles inertes ;
(iii) optionnellement le revêtement des pastilles chargées de rasagiline, des pastilles chargées de pramipexole ou de pastilles chargées à la fois de pramipexole et de rasagiline, obtenues en (ii) avec une sous-couche de revêtement d'isolation/protection ;
(iv) le revêtement des pastilles obtenues en (ii) ou en (iii) avec une couche de revêtement à libération prolongée qui permet une libération prolongée dudit pramipexole et de ladite rasagiline pour ainsi obtenir ladite formulation à libération prolongée ;
(v) optionnellement le mélange des pastilles revêtues obtenues en (iv) avec un excipient approprié ; et
(vi) l'introduction de ladite formulation à libération prolongée dans des capsules ou la compression de ladite formulation à libération prolongée pour obtenir des comprimés, dans lequel lesdites capsules ou lesdits comprimés comprennent un rapport entre les pastilles chargées de pramipexole et les pastilles chargées de rasagiline choisi dans une plage allant de 1 : 1,1 à 1 : 20, de 1 : 1,1 à 1 : 10, de 1 : 1,1 à 1 : 5, de 1 : 1,1 à 1 : 3 ou de 1 : 1,1 à 1 : 2 ; ou lesdites capsules ou lesdits comprimés comprennent des pastilles chargées à la fois avec du pramipexole et de la rasagiline à un rapport choisi dans une plage allant de 1 : 1,1 à 1 : 20, de 1 : 1,1 à 1 : 10, de 1 : 1,1 à 1 : 5, de 1 : 1,1 à 1 : 3 ou de 1 : 1,1 à 1 : 2,
pour ainsi obtenir une formulation à libération prolongée d'une combinaison de doses fixes de pramipexole et de rasagiline.
